# EUROPEAN PATENT APPLICATION

(11) **EP 4 506 006 A1**
(43) Date of publication of application: **12.02.2025**
(21) Application number: 23784324.8
(22) Date of filing: 06.04.2023
(51) Int. Cl.: A61K 31/519, A61K 31/5377, A61K 9/08, A61K 9/10, A61P 17/14, A61P 37/00

(54) **PHARMACEUTICAL COMPOSITION COMPRISING JAK INHIBITOR, PREPARATION METHOD THEREFOR AND USE THEREOF**

(30) Priority: 08.04.2022 CN 202210380702; 22.03.2023 CN 202310326245
(71) Applicant: Beijing Maxinovel Pharmaceuticals Co., Ltd., Beijing 100176 (CN)
(72) Inventor: WANG, Yuguang, Shenzhen, Guangdong 518055 (CN); HU, Haining, Shenzhen, Guangdong 518055 (CN); BI, Lijun, Shenzhen, Guangdong 518055 (CN); ZHANG, Pingjing, Shenzhen, Guangdong 518055 (CN)
(74) Representative: Body, Marine
(86) International application number: PCT/CN2023/086583
(87) International publication number: WO 2023/193761

(57) **Abstract**

Disclosed are a pharmaceutical composition comprising a JAK inhibitor, a preparation method therefor and use of thereof, and particularly disclosed is a pharmaceutical composition comprising a JAK inhibitor. The pharmaceutical composition comprises the following components: 0.1 wt%-3 wt% of the compound represented by formula (I) or the compound represented by formula (II) or a pharmaceutically acceptable salt thereof, 35 wt%-93 wt% of a solvent, and 5 wt%-63 wt% of a transdermal enhancer. When used as a topical liniment, the pharmaceutical composition of the present invention has good permeability and stability.

## Description

The present application claims the right of priorities of Chinese patent application 2022103807021 filed on April 8, 2022 and Chinese patent application 2023103262452 filed on March 22, 2023. The contents of the above Chinese patent applications are incorporated herein by reference in their entireties.

### TECHNICAL FIELD

The present disclosure belongs to the field of medical technology, and specifically relates to a pharmaceutical composition comprising a JAK inhibitor, a preparation method therefor, and a use thereof.

### BACKGROUND

JAK inhibitors can selectively inhibit JAK kinases, blocking the JAK/STAT pathway. The JAK-STAT signaling pathway, discovered in recent years, is a cytokine-stimulated signal transduction pathway involved in many important biological processes such as cell proliferation, differentiation, apoptosis, and immune regulation. Janus kinase is a nonreceptor tyrosine protein kinase with four family members: JAK1, JAK2, TYK2, and JAK3. The first three are widely present in various tissues and cells, while JAK3 is only found in the bone marrow and lymphatic system. Clinically, JAK inhibitors are mainly used to screen therapeutic drugs for hematological diseases, tumors, rheumatoid arthritis, psoriasis, etc.

Currently, multiple JAK inhibitors have entered clinical research. Most JAK inhibitors have poor selectivity and generally inhibit all four kinase family members. Studies by Quintas-Cardama A et al. have shown that selective JAK inhibitors used in clinical trials for rheumatoid arthritis (RA), psoriasis, and other autoimmune diseases can significantly reduce spleen volume, improve clinical symptoms, and enhance the quality of life for patients. Currently, FDA has approved two types of JAK inhibitors: Ruxolitinib, developed by the multinational pharmaceutical company Novartis, and Tofacitinib, developed by Pfizer. Ruxolitinib, which mediates the signal of several cytokines and growth factors important for hematopoietic and immune functions by inhibiting the JAK pathway, is the first approved oral drug specifically for the treatment of myelofibrosis. Tofacitinib is the first approved oral JAK inhibitor for the treatment of RA.

Alopecia areata is a common non-scarring inflammatory alopecia disease, and its pathogenesis and mechanism are not fully understood. The currently recognized mechanism is that alopecia areata is a T-cell-mediated organ-specific autoimmune disease. In patients with alopecia areata, the immune-privileged structure of the anagen hair follicles is disrupted, leading to autoimmune inflammation of the hair follicles, altering the hair follicle cycle, and causing the hair to enter the telogen phase prematurely. Many cytokines involved in the pathogenesis of autoimmune and inflammatory diseases transmit intracellular signals through the JAK/STAT signaling pathway.

Atopic dermatitis (AD) is a pruritic eczematous dermatitis with long-term fluctuations in symptoms with remission and relapse. Currently, topical corticosteroids and calcineurin inhibitors are the main treatments for controlling atopic skin inflammation. However, these drugs have potential safety hazards and have side effects such as serious stimulation of skin atrophy and telangiectasia. The pathology of atopic dermatitis is immunological abnormalities, which manifest as skin barrier dysfunction and pruritus.

Clinical studies have proven that JAK inhibitors have excellent efficacy in autoimmune diseases. However, most of the current JAK inhibitors are oral drugs. Oral drugs are systemic drugs and can cause major side effects. A JAK inhibitor of formula I or a JAK inhibitor of formula II (the compound is further described in patents CN103936757A and WO2014111037A) can bind to JAK proteins, thereby inhibiting JAK phosphorylation, resulting in the obstruction of downstream STAT phosphorylation, blocking the signal transduction of multiple inflammatory cytokines, thereby inhibiting the signal transduction of related factors dependent on the JAK/STAT pathway, such as IFN-γ, IL-6, and IL-15, blocking intracellular signal transduction, inhibiting immune cell activation and T cell-mediated inflammatory diseases.

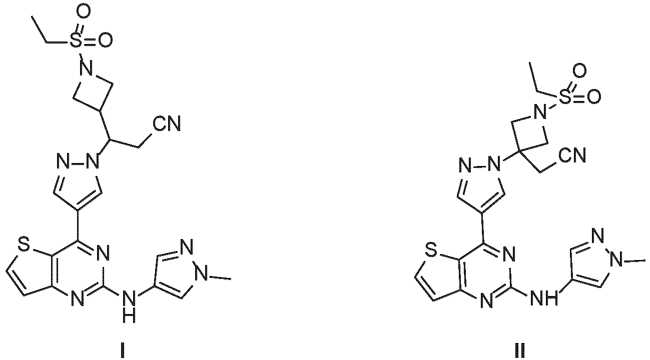

### CONTENT OF THE PRESENT INVENTION

The technical problem to be solved by the present disclosure is to overcome the safety hazards of systemic drug exposure caused by long-term oral administration of JAK inhibitors in the prior art, and to provide a pharmaceutical composition comprising a JAK inhibitor, a preparation method therefor, and a use thereof. The pharmaceutical composition comprising the JAK inhibitor of the present disclosure is for topically used externally and has the advantages of good penetration effect, good stability, and small side effects.

The present disclosure mainly solves the above technical problems through the following technical means.

The present disclosure provides a pharmaceutical composition comprising a JAK inhibitor, which comprises the following components in mass fraction: 0.1% to 3% of a compound of formula I or a pharmaceutically acceptable salt thereof, 35% to 93% of a solvent, and 5% to 63% of a transdermal enhancer;
the solvent is selected from one or more of water, glycerol, polyethylene glycol 400, dimethyl sulfoxide, ethyl acetate, propylene glycol, or ethanol;
the transdermal enhancer is selected from one or more of diethylene glycol monoethyl ether, azone, urea, oleic acid, diisopropyl adipate, menthol, N-methylpyrrolidone, imidurea, isopropyl myristate, cyclodextrin, sodium dodecyl sulfate, polyoxyethylene lauryl ether, polyethylene glycol hexadecyl ether, Tween 20, Tween 40, Tween 60, Tween 80, Span 20, Span 40, Span 60, Span 80, Span 85, deoxycholate, glycocholate, glyceryl monocaprylate, caprylic/capric mono- and diglycerides, propylene glycol monolaurate, propylene glycol monocaprylate, or glyceryl behenate;

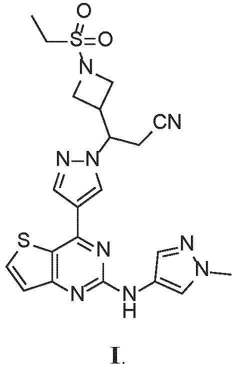

In a certain embodiment of the present disclosure, the pharmaceutical composition comprising the JAK inhibitor comprises the following components in mass fraction: 0.1% to 3% of a compound of formula I or a pharmaceutically acceptable salt thereof, 35% to 93% of a solvent, and 5% to 63% of a transdermal enhancer;
the solvent is selected from one or more of water, glycerol, polyethylene glycol 400, dimethyl sulfoxide, ethyl acetate, propylene glycol, or ethanol;
the transdermal enhancer is selected from one or more of diethylene glycol monoethyl ether, azone, urea, oleic acid, diisopropyl adipate, menthol, N-methylpyrrolidone, imidurea, propyl gallate, isopropyl myristate, cyclodextrin, sodium dodecyl sulfate, polyoxyethylene lauryl ether, polyethylene glycol hexadecyl ether, Tween 20, Tween 40, Tween 60, Tween 80, Span 20, Span 40, Span 60, Span 80, Span 85, deoxycholate, glycocholate, glyceryl monocaprylate, caprylic/capric mono- and diglycerides, propylene glycol monolaurate, propylene glycol monocaprylate, or glyceryl behenate. In a certain embodiment of the present disclosure, the compound of formula I or the pharmaceutically acceptable salt thereof has a mass fraction preferably of 0.3% to 2.5%, more preferably 0.5% to 2%, for example, 0.5% or 2%.

In a certain embodiment of the present disclosure, the solvent has a mass fraction preferably of 38% to 90%; more preferably 40% to 88%, for example, 40%, 45%, 69.3%, 81%, 82.5%, or 87.94%; and particularly preferably 78% to 85%, for example, 81% or 82.5%.

In a certain embodiment of the present disclosure, the transdermal enhancer has a mass fraction preferably of 8% to 60%; more preferably 10% to 58%, for example, 10.06%, 17%, 28.7%, 53%, or 58%; and particularly preferably 15% to 19%, for example, 17%.

In a certain embodiment of the present disclosure, the solvent is preferably selected from one or more of polyethylene glycol 400, dimethyl sulfoxide, propylene glycol, or ethanol, for example, polyethylene glycol 400; a mixed solvent of polyethylene glycol 400 and dimethyl sulfoxide; a mixed solvent of polyethylene glycol 400, propylene glycol, and ethanol; or a mixed solvent of polyethylene glycol 400, propylene glycol, and dimethyl sulfoxide.

In a certain embodiment of the present disclosure, the solvent is more preferably selected from one or more of polyethylene glycol 400, dimethyl sulfoxide, or propylene glycol, and is particularly preferably a mixed solvent of polyethylene glycol 400, dimethyl sulfoxide, and propylene glycol.

In a certain embodiment of the present disclosure, the transdermal enhancer is preferably selected from one or more of diethylene glycol monoethyl ether, azone, oleic acid, diisopropyl adipate, menthol, N-methylpyrrolidone, imidurea, or propyl gallate, for example, a mixed transdermal enhancer of diethylene glycol monoethyl ether, propyl gallate, and oleic acid; a mixed transdermal enhancer of diethylene glycol monoethyl ether, propyl gallate, and diisopropyl adipate; a mixed transdermal enhancer of diethylene glycol monoethyl ether, propyl gallate, and menthol; a mixed transdermal enhancer of diethylene glycol monoethyl ether, propyl gallate, and N-methylpyrrolidone; a mixed transdermal enhancer of diethylene glycol monoethyl ether, propyl gallate, and imidurea; a mixed transdermal enhancer of diethylene glycol monoethyl ether and azone; a mixed transdermal enhancer of diethylene glycol monoethyl ether and oleic acid; or a mixed transdermal enhancer of diethylene glycol monoethyl ether and menthol.

In a certain embodiment of the present disclosure, the transdermal enhancer is more preferably selected from one or two of diethylene glycol monoethyl ether or oleic acid, and is particularly preferably a mixed transdermal enhancer of diethylene glycol monoethyl ether and oleic acid.

In a certain embodiment of the present disclosure, when the solvent is a mixed solvent of polyethylene glycol 400 and dimethyl sulfoxide, the mass ratio of polyethylene glycol 400 to dimethyl sulfoxide is preferably from 6:1 to 10:1, more preferably from 7:1 to 9:1, for example, 8:1.

In a certain embodiment of the present disclosure, when the solvent is a mixed solvent of polyethylene glycol 400, propylene glycol, and ethanol, the mass ratio of polyethylene glycol 400 to propylene glycol is preferably from 2:1 to 6:1, more preferably from 3:1 to 5:1, for example, 4:1. The mass ratio of propylene glycol to ethanol is preferably from 1:1 to 1:3, more preferably from 1:1.5 to 1:2.5, for example, 1:2.

In a certain embodiment of the present disclosure, when the solvent is a mixed solvent of polyethylene glycol 400, propylene glycol, and dimethyl sulfoxide, the mass ratio of polyethylene glycol 400 to dimethyl sulfoxide may be from 3:1 to 10:1. The mass ratio of propylene glycol to dimethyl sulfoxide may be from 3:1 to 9:1.

In a certain embodiment of the present disclosure, when the solvent is a mixed solvent of polyethylene glycol 400, propylene glycol, and dimethyl sulfoxide, the mass ratio of polyethylene glycol 400 to dimethyl sulfoxide is preferably from 6:1 to 10:1, more preferably from 7:1 to 9:1, for example, 8:1 or 8.3:1. The mass ratio of propylene glycol to dimethyl sulfoxide is preferably from 5:1 to 9:1, more preferably from 6:1 to 8:1, for example, 7.2:1.

In a certain embodiment of the present disclosure, when the solvent is a mixed solvent of polyethylene glycol 400, propylene glycol, and dimethyl sulfoxide, the mass ratio of polyethylene glycol 400 to dimethyl sulfoxide may be 4:1. The mass ratio of propylene glycol to dimethyl sulfoxide may be 3.794:1.

In a certain embodiment of the present disclosure, when the transdermal enhancer is a mixed transdermal enhancer of diethylene glycol monoethyl ether, propyl gallate, and oleic acid, the mass ratio of diethylene glycol monoethyl ether to oleic acid is preferably from 2:1 to 5:1, more preferably from 3:1 to 4:1, for example, 3.57:1. The mass ratio of propyl gallate to oleic acid is preferably from 2:1 to 5:1, more preferably from 3:1 to 4:1, for example, 3.71:1.

In a certain embodiment of the present disclosure, when the transdermal enhancer is a mixed transdermal enhancer of diethylene glycol monoethyl ether, propyl gallate, and diisopropyl adipate, the mass ratio of diethylene glycol monoethyl ether to diisopropyl adipate is preferably from 2:1 to 5:1, more preferably from 3:1 to 4:1, for example, 3.57:1. The mass ratio of propyl gallate to diisopropyl adipate is preferably from 2:1 to 5:1, more preferably from 3:1 to 4:1, for example, 3.71:1.

In a certain embodiment of the present disclosure, when the transdermal enhancer is a mixed transdermal enhancer of diethylene glycol monoethyl ether, propyl gallate, and menthol, the mass ratio of diethylene glycol monoethyl ether to menthol is preferably from 150:1 to 430:1, more preferably from 160:1 to 420:1, for example, 166.67:1 or 416.67:1. The mass ratio of propyl gallate to menthol is preferably from 530:1 to 730:1, more preferably from 540:1 to 720:1, for example, 549:1 or 715.67:1.

In a certain embodiment of the present disclosure, when the transdermal enhancer is a mixed transdermal enhancer of diethylene glycol monoethyl ether, propyl gallate, and *N-*methylpyrrolidone, the mass ratio of diethylene glycol monoethyl ether to *N-*methylpyrrolidone is preferably from 2:1 to 5:1, more preferably from 3:1 to 4:1, for example, 3.57:1. The mass ratio of propyl gallate to N-methylpyrrolidone is preferably from 2:1 to 5:1, more preferably from 3:1 to 4:1, for example, 3.71:1.

In a certain embodiment of the present disclosure, when the transdermal enhancer is a mixed transdermal enhancer of diethylene glycol monoethyl ether, propyl gallate, and imidurea, the mass ratio of diethylene glycol monoethyl ether to imidurea is preferably from 610:1 to 640:1, more preferably from 620:1 to 630:1, for example, 625:1. The mass ratio of propyl gallate to imidurea is preferably from 810:1 to 840:1, more preferably from 820:1 to 830:1, for example, 824:1.

In a certain embodiment of the present disclosure, when the transdermal enhancer is a mixed transdermal enhancer of diethylene glycol monoethyl ether and azone, the mass ratio of diethylene glycol monoethyl ether to azone is preferably from 4:1 to 8:1, more preferably from 5:1 to 7:1, for example, 6.27:1.

In a certain embodiment of the present disclosure, when the transdermal enhancer is a mixed transdermal enhancer of ethylene glycol monoethyl ether and oleic acid, the mass ratio of ethylene glycol monoethyl ether to oleic acid is preferably from 1.2:1 to 1.6:1, more preferably from 1.3:1 to 1.5:1, for example, 1.43:1.

In a certain embodiment of the present disclosure, when the transdermal enhancer is a mixed transdermal enhancer of diethylene glycol monoethyl ether and menthol, the mass ratio of diethylene glycol monoethyl ether to menthol is preferably from 150:1 to 180:1, more preferably from 160:1 to 170:1, for example, 166.67:1.

In a certain embodiment of the present disclosure, the pharmaceutical composition comprising the JAK inhibitor is composed of the 0.1% to 3% of the compound of formula I or the pharmaceutically acceptable salt thereof, 35% to 93% of the solvent, and 5% to 63% of the transdermal enhancer.

In a certain embodiment of the present disclosure, the pharmaceutical composition comprising the JAK inhibitor is preferably composed of components shown in any one of the following schemes:
Scheme 1: 2% of the compound of formula I or the pharmaceutically acceptable salt thereof, 40% of polyethylene glycol 400, 25% of diethylene glycol monoethyl ether, 26% of propyl gallate, and 7% of oleic acid.
Scheme 2: 2% of the compound of formula I or the pharmaceutically acceptable salt thereof, 40% of polyethylene glycol 400, 25% of diethylene glycol monoethyl ether, 26% of propyl gallate, and 7% of diisopropyl adipate.
Scheme 3: 2% of the compound of formula I or the pharmaceutically acceptable salt thereof, 40% of polyethylene glycol 400, 25% of diethylene glycol monoethyl ether, 32.94% of propyl gallate, and 0.06% of menthol.
Scheme 4: 2% of the compound of formula I or the pharmaceutically acceptable salt thereof, 40% of polyethylene glycol 400, 25% of diethylene glycol monoethyl ether, 26% of propyl gallate, and 7% of N-methylpyrrolidone.
Scheme 5: 2% of the compound of formula I or the pharmaceutically acceptable salt thereof, 40% of polyethylene glycol 400, 10% of diethylene glycol monoethyl ether, 42.94% of propyl gallate, 0.06% of menthol, and 5% of dimethyl sulfoxide.
Scheme 6: 2% of the compound of formula I or the pharmaceutically acceptable salt thereof, 40% of polyethylene glycol 400, 25% of diethylene glycol monoethyl ether, 32.96% of propyl gallate, and 0.04% of imidurea.
Scheme 7: 2% of the compound of formula I or the pharmaceutically acceptable salt thereof, 39.6% of polyethylene glycol 400, 24.75% of diethylene glycol monoethyl ether, 9.9% of propylene glycol, 19.8% of ethanol, and 3.95% of azone.
Scheme 8: 2% of the compound of formula I or the pharmaceutically acceptable salt thereof, 40% of polyethylene glycol 400, 10% of diethylene glycol monoethyl ether, 36% of propylene glycol, 7% of oleic acid, and 5% of dimethyl sulfoxide.
Scheme 9: 0.5% of the compound of formula I or the pharmaceutically acceptable salt thereof, 41.5% of polyethylene glycol 400, 10% of diethylene glycol monoethyl ether, 36% of propylene glycol, 7% of oleic acid, and 5% of dimethyl sulfoxide.
Scheme 10: 2% of the compound of formula I or the pharmaceutically acceptable salt thereof, 40% of polyethylene glycol 400, 10% of diethylene glycol monoethyl ether, 37.94% of propylene glycol, 0.06% of menthol, and 10% of dimethyl sulfoxide.
Scheme 11: 2% of the compound of formula I or the pharmaceutically acceptable salt thereof, 40% of polyethylene glycol 400, 25% of diethylene glycol monoethyl ether, 32.96% of propyl gallate, and 0.06% of imidurea. Scheme 12: 2% of the compound of formula I or the pharmaceutically acceptable salt thereof, 40% of polyethylene glycol 400, 25% of diethylene glycol monoethyl ether, 32.96% of propyl gallate, and 3.96% of imidurea.

Percentages above are all mass percentages.

In a certain embodiment of the present disclosure, in the above schemes 1 to 10, the compound of formula I or the pharmaceutically acceptable salt thereof is preferably the compound of formula I. In a certain embodiment of the present disclosure, in the above schemes 1 to 12, the compound of formula I or the pharmaceutically acceptable salt thereof is preferably the compound of formula I.

In a certain embodiment of the present disclosure, the pharmaceutical composition comprising the JAK inhibitor is more preferably composed of components shown in scheme 8 or scheme 9.

In a certain embodiment of the present disclosure, the pharmaceutical composition may be formulated into an ointment, a gel, a solution, a spray, or a suspension, preferably a solution.

In each embodiment of the above pharmaceutical composition comprising the JAK inhibitor, the compound of formula I can be replaced by a compound of formula II;

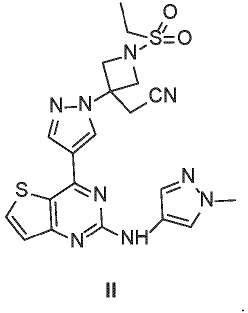

The present disclosure further provides a preparation method for the pharmaceutical composition comprising the JAK inhibitor, and the preparation method may comprise the following step: mixing the compound of formula I or the pharmaceutically acceptable salt thereof, the solvent, and the transdermal enhancer.

In a certain embodiment of the present disclosure, the preparation method for the pharmaceutical composition comprising the JAK inhibitor preferably comprises the following steps: after mixing the solvent and the transdermal enhancer, dissolving the compound of formula I or the pharmaceutically acceptable salt thereof therein, to obtain the pharmaceutical composition comprising the JAK inhibitor. The compound of formula I or the pharmaceutically acceptable salt thereof is preferably the compound of formula I.

In a certain embodiment of the present disclosure, the dissolving may be by means of ultrasound, heating, or a combination of both. The heating is preferably water bath heating. The heating is preferably at a temperature of 60°C to 70°C.

In a certain embodiment of the present disclosure, when the pharmaceutical composition comprising the JAK inhibitor comprises dimethyl sulfoxide (for example, when the pharmaceutical composition is scheme 5, scheme 8, scheme 9, or scheme 10), the preparation method for the pharmaceutical composition preferably comprises the following steps:
(1) mixing the dimethyl sulfoxide and the compound of formula I or the pharmaceutically acceptable salt thereof in the components to obtain a mixture;
(2) mixing all components except the dimethyl sulfoxide and the compound of formula I or the pharmaceutically acceptable salt thereof in the components to obtain a mixture;
(3) mixing the mixture of step (1) with the mixture of step (2) to obtain the pharmaceutical composition comprising the JAK inhibitor.

Preferably, the compound of formula I or the pharmaceutically acceptable salt thereof is the compound of formula I.

Preferably, in step (3), the mixing is adding the mixture of step (2) to the mixture of step (1).

Preferably, the mixture of step (1) and the mixture of step (2) are each mixed homogeneously by stirring. The stirring is preferably at a rotation speed of 1500 rpm to 2500 rpm, for example, 2000 rmp.

In a certain embodiment of the present disclosure, the prepared pharmaceutical composition comprising the JAK inhibitor is preferably stored at room temperature away from light.

In each embodiment of the above preparation method for the pharmaceutical composition comprising the JAK inhibitor, the compound of formula I can be replaced by a compound of formula II;

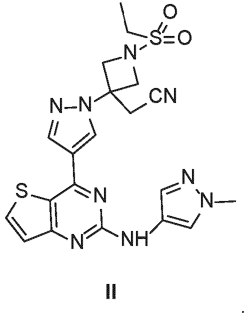

The present disclosure further provides a topical liniment, which may comprise the pharmaceutical composition comprising the JAK inhibitor.

The present disclosure further provides a use of the pharmaceutical composition comprising the JAK inhibitor or the topical liniment for preventing, alleviating, or treating skin autoimmune diseases related to JAK kinase.

In a certain embodiment of the present disclosure, the skin autoimmune disease is preferably selected from one or more of alopecia areata, atopic dermatitis, or psoriasis.

In the present disclosure, the solvent can promote the dissolution of an active ingredient. The transdermal enhancer can promote the penetration of the active ingredient. In some cases, the solvent can also promote the penetration of the active ingredient. The transdermal enhancer can also promote the dissolution of the active ingredient.

In the present disclosure, the term "pharmaceutically acceptable salt" refers to a pharmaceutically acceptable organic or inorganic salt of the compound of the present disclosure. Exemplary salts include, but are not limited to: sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethane sulfonate, benzenesulfonate, p-toluenesulfonate, and pamoate (i.e., 1-1-methylene-bis(2-hydroxy-3-naphthoate)).

In accordance with common knowledge in the art, the above various preferred conditions can be arbitrarily combined to obtain the various preferred embodiments of the present disclosure.

The reagents and raw materials used in the present disclosure are commercially available.

The positive and progressive effect of the present disclosure is that the present disclosure provides a new pharmaceutical composition for topical liniment comprising a JAK inhibitor, and the pharmaceutical composition has a wide safety window, good permeability and stability.

### BRIEF DESCRIPTION OF THE DRAWINGS

FIG. 1 shows the average cumulative permeation rates of formulations C, D, and E with 2% (w/w) specifications in an *in vitro* permeation test;
FIG. 2 shows the average cumulative permeation rates of formulations F11, A2, and E2 with 2% (w/w) specifications in an *in vitro* permeation test.

### DETAILED DESCRIPTION OF THE PREFERRED EMBODIMENT

The present disclosure is further described through examples below, but is not limited to the scope of the examples provided. In the following examples, experimental methods without specified conditions are carried out according to conventional methods and conditions, or are selected according to the product instructions.

### Example 1: Saturation solubility test of the compound of formula I in different solvents

**Table 1**

| Solvent | Solubility (mg/mL) |
|---|---|
| Dimethyl sulfoxide | 302.13 |
| Polyethylene glycol 400 | 26.65 |
| Diethylene glycol monoethyl ether | 14.04 |
| Ethyl acetate | 2.34 |
| Propylene glycol | 0.99 |
| Anhydrous ethanol | 0.24 |
| Oleic acid | 0.23 |
| Glycerol | 0.07 |
| Medium chain triglycerides | 0.075 |
| Soybean oil | 0.018 |
| Corn oil | 0.014 |
| Olive oil | 0.014 |
| pH2 phosphate buffer | 0.016 |
| pH4 phosphate buffer | 0.001 |
| pH6 phosphate buffer | 0.001 |
| pH8 phosphate buffer | 0.001 |
| Pure water | 0.001 |

Example 2: Preparation of a pharmaceutical composition containing 2% (w/w) of the compound of formula I

**Table 2**

| **Component** | **Formulation A** | **Formulation B** | **Formulation C** | **Formulation D** | **Formulation E** | **Formulation H** |
|---|---|---|---|---|---|---|
| **Compound of formula I** | 2.00% | 2.00% | 2.00% | 2.00% | 2.00% | 2.00% |
| **Polyethylene glycol 400** | 40.00% | 40.00% | 40.00% | 40.00% | 40.00% | 40.00% |
| **Diethylene glycol monoethyl ether** | 25.00% | 25.00% | 25.00% | 25.00% | 10.00% | 25.00% |
| **Propyl gallate** | 26.00% | 26.00% | 32.94% | 26.00% | 42.94% | 32.96% |
| **Ethanol** | - | - | - | - | - | - |
| **Azone** | - | - | - | - | - | - |
| **Oleic acid** | 7.00% | - | - | - | - | - |
| **Diisopropyl adipate** | - | 7.00% | - | - | - | - |
| **Menthol** | - | - | 0.06% | - | 0.06% | - |
| ***N-*Methylpyrrolidone** | - | - | - | 7.00% | - | - |
| **Dimethyl sulfoxide** | - | - | - | - | 5.00% | - |
| **Imidurea** | - | - | - | - | - | 0.04% |
| **Total** | 100.00% | 100.00% | 100.00% | 100.00% | 100.00% | 100.00% |

### Preparation method:

(1) According to the proportions in Table 2, 9.8 g of blank prescriptions A, B, C, D, E, and H were prepared. After weighing, the above prescriptions were vigorously shaken and ultrasonicated to mix uniformly.
(2) Six portions of 0.1 g of the active ingredient compound of formula I were weighed into clean vials with lids, and 4.9 g of the blank prescriptions prepared above were added to each vial.
(3) The samples were continuously ultrasonicated at room temperature for 30 minutes. The samples did not dissolve completely, and the samples were heated in a 70°C water bath until completely dissolved and clear. The samples were then stored at room temperature away from light for later use.

### Example 3: Preparation of a pharmaceutical composition containing 2% (w/w) of the compound of formula I

**Table 3**

| **Component** | **Formulation F11** | **Formulation A2** | **Formulation E2** |
|---|---|---|---|
| **Compound of formula I** | 2.00% | 2.00% | 2.00% |
| **Polyethylene glycol 400** | 39.60% | 40.00% | 40.00% |
| **Diethylene glycol monoethyl ether** | 24.75% | 10.00% | 10.00% |
| **Propylene glycol** | 9.90% | 36.00% | 37.94% |
| **Ethanol** | 19.80% | - | - |
| **Azone** | 3.95% | - | - |
| **Oleic acid** | - | 7.00% | - |
| **Menthol** | - | - | 0.06% |
| **Dimethyl sulfoxide** | - | 5.00% | 10.00% |
| **Total** | 100.00% | 100.00% | 100.00% |

### Preparation method:

(1) According to the proportions in Table 3, 9.8 g of blank prescriptions F11, A2, and E2 were prepared. After weighing, the above prescriptions were vigorously shaken and ultrasonicated to mix uniformly.
(2) Three portions of 0.1 g of the active ingredient compound of formula I were weighed into clean vials with lids, and 4.9 g of the blank prescriptions prepared above were added to each vial.
(3) After ultrasonicating 30 minutes, prescription E2 was dissolved completely. Prescriptions F11 and A2 were further heated in a 70°C water bath for 5 minutes, then ultrasonicated for 2 minutes, prescription F11 was dissloved completely. Prescription A2 was further heated for 2 minutes until completely dissolved. The samples were stored at room temperature away from light for later use.

### Example 4: In vitro penetration test (IVPT)

### (1) Pre-experimental preparation

The transdermal diffusion apparatus was turned on and set with the parameters: temperature at 32°C and rotation speed at 600 rpm. The clean diffusion receptor cells corresponding to the number of prescriptions were selected and numbered, and the calibrated volumes on the bottles were recorded. A piece of rat skin was preferably selected to conduct the diffusion cell experiment in parallel corresponding to the number of prescriptions. The skin was removed while still cold, shaved, and circular pieces of the corresponding number of prescriptions of rat skin similar in outer diameter to the supply chamber were cut. The rat skin was then fixed with a clamp, and the blank receptor solution (0.1× PBS) was added. During the adding process, care was taken to remove air bubbles. The receptor cells of the corresponding number of prescriptions were sequentially placed into the transdermal diffusion apparatus, and the temperature was maintained at 32°C for 30 minutes to allow the temperature of the blank receptor solution to reach 32°C before the experiment was started.

### (2) Experimental operation

The corresponding numbered formulation prototype prescription solutions were added dropwise onto each receptor cell, with 20 µL of each prescription being added, and were evenly spread using a pipette tip. The state of the drug solution on the skin at the upper mouth of each diffusion cell was observed throughout the experiment. Sampling times were set at 18 hours, 24 hours, 36 hours, and 48 hours. Five minutes before each sampling point, the blank receptor cell solution was replenished to the mouth of the receptor cells, and the solutions in each diffusion receptor cell were rinsed using the corresponding numbered syringes and a 16-gauge rat gavage needle. After rinsing was completed, 2 mL of samples were collected and placed into 5 mL centrifuge tubes, which were then numbered. The centrifugation conditions were set to 12,000 rpm for 10 minutes at room temperature. After centrifugation, the supernatant was collected and prepared for liquid phase detection. After the sampling and centrifugation were completed, the blank receptor cell solution was replenished to the sampling ports of each diffusion receptor cell, filling to the mouth of the cells. The transdermal diffusion of the formulation was observed, and no further replenishment was made after the 48-hour sampling. The apparatus was then turned off.

After sampling and centrifugation at each time point, liquid phase detection was conducted. The liquid phase detection and analysis methods are as follows:

**Table 4**

| **Analytical method** | |
|---|---|
| **Chromatographic column** | Agilent Eclipse Plus C18 100 * 4.6 mm, 5.0 µm, chromatographic column number: LC-F-046 |
| **Mobile phase A** | 30 mM ammonium acetate aqueous solution: acetonitrile = 19:1 |
| **Mobile phase B** | Acetonitrile |
| **Blank diluent** | Water: acetonitrile = 6:4 |
| **Isocratic ratio** | Mobile phase A: mobile phase B = 65:35 |
| **Running time per injection** | 10 min |
| **Post-running time** | None |
| **Needle washing method** | 5 µL of blank diluent |
| **Flow rate** | 1.0 mL/min |
| **Detection wavelength** | 275 nm |
| **Injection volume** | 100 µL |
| **Column temperature** | 35°C |

### (3) Post-experiment rat skin processing

After the receptor cell solutions were completely drained, each numbered diffusion receptor cell device was washed with a blank diluent (acetonitrile: water = 4:1) to remove any residual drug solution from the rat skin. The rat skin from each numbered diffusion cell was respectively removed, and the shaved parts were dried with coarse filter paper. The part in contact with the receptor cell was rinsed with water, dried with coarse filter paper, and placed in the corresponding homogenization tube with a labeled number. The weight of each tube was recorded, and the samples were stored at -20°C in a refrigerator before undergoing LC-MS analysis.

### (4) Receptor cell sampling test results

The average cumulative permeation rate of six formulations of C, D, E, F11, A2, and E2 with 2% (w/w) specifications at each sampling point was measured.

### (5) Experimental results

**Table 5: Average cumulative permeation rates at each sampling time point in an in vitro permeation test of formulations C, D, and E with 2% (w/w) specifications**

| **Formulation** | **Sampling time point (h)** | **Average cumulative permeation rate%** |
|---|---|---|
| **C** | 18 | 0.010% |
| | 24 | 0.021% |
| | 36 | 0.037% |
| | 48 | 0.042% |
| **D** | 18 | 0.071% |
| | 24 | 0.136% |
| | 36 | 0.275% |
| | 48 | 0.299% |
| **E** | 18 | 0.061% |
| | 24 | 0.095% |
| | 36 | 0.187% |
| | 48 | 0.216% |

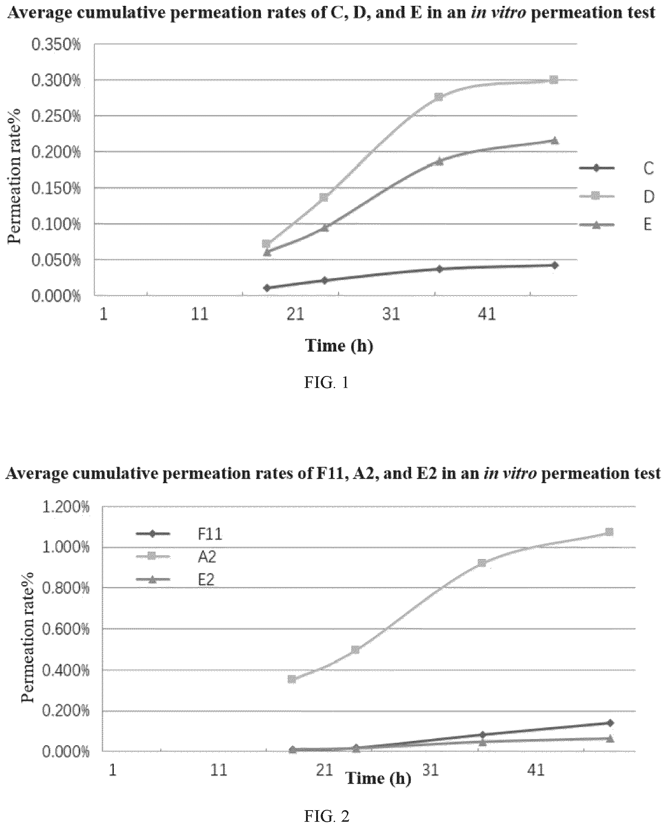

According to the results in FIG. 1 and Table 5, it can be seen that the average cumulative permeation rates at each sampling time point in the *in vitro* permeation test of formulations C, D, and E are in the order of D > E > C from large to small.

**Table 6: Average cumulative permeation rates at each sampling time point in an in vitro permeation test of formulations F11, A2, and E2 with 2% (w/w) specifications**

| **Formulation** | **Sampling time point (h)** | **Average cumulative permeation rate%** |
|---|---|---|
| **F11** | 18 | 0.012% |
| | 24 | 0.020% |
| | 36 | 0.083% |
| | 48 | 0.139% |
| **A2** | 18 | 0.349% |
| | 24 | 0.494% |
| | 36 | 0.919% |
| | 48 | 1.069% |
| **E2** | 18 | 0.010% |
| | 24 | 0.015% |
| | 36 | 0.048% |
| | 48 | 0.065% |

According to the results in FIG. 2 and Table 6, it can be seen that the average cumulative permeation rate of formulation A2 in the *in vitro* permeation test within 48 hours is significantly higher than that of formulations E2 and F11.

### Example 5: Stability test of formulations A2 and E

### (1) Preparation of stability samples of formulation A2 and E solutions

**Table 6**

| **Component** | **A2 (w/w%)** | **E (w/w%)** | **Blank A2 (g)** | **Blank E (g)** | **Drug-containing A2 (g)** | **Drug-containing E (g)** |
|---|---|---|---|---|---|---|
| **Compound of formula I** | 2.00 | 2.00 | NA | NA | 1.47 | 1.47 |
| **Polyethylene glycol 400** | 40.00 | 40.00 | 60 | 60 | 72.03 | 72.03 |
| **Diethylene glycol monoethyl ether** | 10.00 | 10.00 | 15 | 15 | | |
| **Propylene glycol** | 36.00 | 42.94 | 54 | 64.41 | | |
| **Oleic acid** | 7.00 | NA | 10.5 | NA | | |
| **Menthol** | NA | 0.06 | NA | 0.09 | | |
| **Dimethyl sulfoxide** | 5.00 | 5.00 | 7.5 | 7.5 | | |
| **Total** | 100 | 100 | 147 | 147 | 73.5 | 73.5 |

Preparation of blank prescription: Each excipient was weighed according to the prescription amount and placed in a clean glass bottle with a lid. The mixture was stirred magnetically at room temperature for 30 minutes until the excipients were completely dissolved, to obtain a clear and transparent liquid, which was set aside for later use.

Preparation of drug-containing prescription: The active ingredient, the compound of formula I, was accurately weighed into a clean vial. The blank solution prepared in the previous step was added according to the prescribed ratio. The mixture was stirred magnetically in a 70°C water bath for 40 minutes, followed by 10 minutes of ultrasonic treatment to completely dissolve the active ingredient compound of formula I, to obtain a clear and transparent yellow solution.

### (2) Stability sample placement protocol for formulation A2 and E solutions:

**Table 7**

| | | |
|---|---|---|
| Stability sample placement condition | 40°C, 75%RH | 25°C, 60%RH |
| Packaging material | Transparent, brown | Transparent, brown |
| Placement time point (excluding day 0) | Month 4, Month 6 | Day 7, Month 4, Month 6, Month 12 |
| Number of prescriptions | 2 (Formulations A2, E) | 2 (Formulations A2, E) |

### (3) Stability results for formulation A2, E solutions:

**Table 8**

| **Item** | **Test item** | **Time** | | | | |
|---|---|---|---|---|---|---|
| | | **0d** | **7d** | **4M** | **6M** | **12M** |
| **A2-Transparent (25°C, 60%RH)** | Test | 100% | 97% | 96% | 99% | 99% |
| | Related substance | RRT(1.14): 0.03% | RRT(1.14): 0.02% | RRT(0.94): 0.04%; | RRT(0.94): 0.04%; | RRT(0.94): 0.11%; |
| | | | | RRT(0.98): 0.04%; | RRT(0.98): 0.04%; | |
| | | | | | | RRT(0.98): 0.16% |
| | | | | RRT(1.14): 0.03% | RRT(1.14): 0.03% | |
| **A2-Brown (25°C, 60%RH)** | Test | 100% | 97% | 95% | 99% | 97% |
| | Related substance | RRT(1.14): 0.03% | RRT(1.14): 0.02% | RRT(0.94): 0.03%; | RRT(0.94): 0.05%; | RRT(0.94): 0.10%; |
| | | | | RRT(0.98): 0.02%; | RRT(0.98): 0.04%; | |
| | | | | | | RRT(0.98): 0.14% |
| | | | | RRT(1.13): 0.02% | RRT(1.13): 0.03% | |
| **A2-Transparent (40°C, 75%RH)** | Test | 100% | NA | 96% | 99% | NA |
| | Related substance | RRT(1.14): 0.03% | | RRT(0.94): 0.09%; | RRT(0.94): 0.10%; | |
| | | | | RRT(0.98): 0.10%; | | |
| | | | | | RRT(0.98): 0.10%; | |
| | | | | RRT(1.06): 0.01%; | | |
| | | | | | RRT(1.14): 0.03% | |
| | | | | RRT(1.14): 0.03% | | |
| **A2-Brown (40°C, 75%RH)** | Test | 100% | NA | 95% | 100% | NA |
| | Related substance | RRT(1.14): 0.03% | | RRT(0.94): 0.07%; | RRT(0.94): 0.09%; | |
| | | | | RRT(0.98): 0.05%; | | |
| | | | | | RRT(0.98): 0.10%; | |
| | | | | RRT(1.06): 0.02%; | | |
| | | | | | RRT(1.14): 0.03% | |
| | | | | RRT(1.14): 0.03% | | |
| **E-Transparent (25°C, 60%RH)** | Test | 100% | 99% | 98% | 98% | 96% |
| | Related substance | RRT(1.14): 0.03% | RRT(1.14): 0.02% | RRT(1.13): 0.01% | RRT(1.14): 0.01% | Not detected |
| **E-Brown (25°C, 60%RH)** | Test | 100% | 102% | 95% | 100% | 99% |
| | Related substance | RRT(1.14): 0.03% | RRT(1.14): 0.02% | RRT(1.13): 0.01% | RRT(1.14): 0.01% | Not detected |
| **E-Transparent (40°C, 75%RH)** | Test | 100% | NA | 98% | 98% | NA |
| | Related substance | RRT(1.14): 0.03% | | RRT(0.94): 0.03%; | RRT(0.94): 0.03%; | |
| | | | | RRT(0.98): 0.04%; RRT(1.14): 0.02% | RRT(0.98): 0.06%; RRT(1.14): 0.03% | |
| **E-Brown (40°C, 75%RH)** | Test | 100% | NA | 97% | 98% | NA |
| | Related substance | RRT(1.14): 0.03% | | RRT(0.94): 0.03%; | RRT(0.94): 0.03%; | |
| | | | | RRT(0.98): 0.04%; | RRT(0.98): 0.06%; | |
| | | | | RRT(1.14): 0.03% | RRT(1.14): 0.02% | |

Experiments have shown that formulation A2 and formulation E are very stable, and there is no significant difference in the stability between transparent bottles and brown bottles.

### Example 6: Preparation process of 2% (w/w) specification of formulation A2

### (1) Prescription composition of 2% (w/w) specification of formulation A2

**Table 9**

| **Material** | **Ratio% (w/w)** | **Prescription amount (g)** |
|---|---|---|
| Compound of formula I | 2 | 37 |
| Polyethylene glycol 400 | 40 | 740 |
| Diethylene glycol monoethyl ether | 10 | 185 |
| Propylene glycol | 36 | 666 |
| Oleic acid | 7 | 129.5 |
| Dimethyl sulfoxide | 5 | 92.5 |
| **Total** | 100 | 1850 |

### (2) Preparation steps

### a. Preparation of intermediate 1

**Table 10**

| **Material** | **Prescription amount (g)** |
|---|---|
| Compound of formula I | 37 |
| Dimethyl sulfoxide | 92.5 |
| **Total** | 129.5 |

The prescription amount of the active pharmaceutical ingredient compound of formula I was weighed and dissolved completely in the prescription amount of dimethyl sulfoxide by stirring at 2000 rpm for 5 minutes to obtain intermediate 1, which was set aside for later use.

### b. Preparation of mixed solvent

**Table 11**

| **Material** | **Weighing amount (g)** |
|---|---|
| Polyethylene glycol 400 | 740 |
| Diethylene glycol monoethyl ether | 185 |
| Propylene glycol | 666 |
| Oleic acid | 129.5 |
| **Total** | 1720.5 |

According to the ratios in the above table, the remaining excipients were weighed into another container and stirred at 2000 rpm for 5 minutes to be fully mixed, to obtain a mixed solvent for later use.

### c. Preparation of intermediate 2

The mixed solvent from step b was transferred to intermediate 1 from step a, and stirred at 2000 rpm for about 10 minutes to be fully mixed, to obtain intermediate 2.

### d. Intermediate control

### Homogeneity detection:

Samples were taken from the upper, middle, and lower layers of intermediate 2 solution, with three points sampled from different positions within each layer. Approximately 1 g of sample was taken from each point to detect the homogeneity and calculate the RSD value.

### Content detection:

Approximately 1 g of sample was taken from the upper, middle, and lower layers of intermediate 2 solution and mixed into a single sample to detect the content in the solution.

### e. Filling

After the process control results met the predetermined quality standards, intermediate 2 was filled into tubular glass injection vials (brown) made of middle borosilicate using a liquid filling machine. The filling machine was adjusted to ensure a fill volume of approximately 3.7 g/vial, with a fill range of 3.7 g/vial to 4.07 g/vial. The vials were sealed with brominated butyl rubber stoppers for injectable solutions and aluminum-plastic combination caps for antibiotic vials.

### Example 7: Preparation process of 0.5% (w/w) specification of formulation A2

### (1) Prescription composition of 0.5% (w/w) specification of formulation A2

**Table 12**

| **Material** | **Ratio% (w/w)** | **Prescription amount (g)** |
|---|---|---|
| Compound of formula I | 0.5 | 13.25 |
| Polyethylene glycol 400 | 41.5 | 1099.75 |
| Diethylene glycol monoethyl ether | 10.0 | 265.0 |
| Propylene glycol | 36.0 | 954.0 |
| Oleic acid | 7.0 | 185.5 |
| Dimethyl sulfoxide | 5.0 | 132.5 |
| **Total** | 100.0 | 2650 |

### (2) Preparation steps

According to the prescription composition of the 0.5% (w/w) specification of formulation A2, the preparation was completed with reference to the preparation steps of Example 6.

### Example 8: Stability study of 2% (w/w) specification of formulation A2

**Table 13**

| Placement condition | Test item | Day 0 | Day 5 | Day 10 | Day 30 |
|---|---|---|---|---|---|
| Illumination (4500 Lx ± 500 Lx) | Content of compound of formula I | 99.3% | 100.2% | 99.0% | 99.6% |
| | Content of related substances (total impurity) | Not detected | Not detected | Not detected | Not detected |
| **High temperature** (60 ± 2°C) | Content of compound of formula I | 98.3% | 101.4% | 100.5% | 101.3% |
| | Content of related substances (total impurity) | Not detected | Not detected | Not detected | Not detected |
| High humidity (92.5% RH) | Content of compound of formula I | 98.3% | 101.4% | 100.5% | 101.3% |
| | Content of related substances (total impurity) | Not detected | Not detected | Not detected | Not detected |

### Example 9: Accelerated stability test of 2% (w/w) specification of formulation A2

**Table 14**

| Test item | Placement condition | Day 0 | Month 1 | Month 2 | Month 3 |
|---|---|---|---|---|---|
| Property | 40 ± 2°C, 75 ± 5%RH | Yellow transpare nt oily liquid | Yellow transpare nt oily liquid | Yellow transparen t oily liquid | Yellow transpare nt oily liquid |
| Content of compound of formula I | | 99.3% | 100.0% | 100.0% | 99.3% |
| Related substance (total impurity content) | | Not detected | Not detected | 0.06% | Not detected |

### Example 10: Stability study of 0.5% (w/w) specification of formulation A2

**Table 15**

| Placement condition | Test item | Day 0 | Day 5 | Day 10 | Day 30 |
|---|---|---|---|---|---|
| Illumination (4500 Lx ± 500 Lx) | Content of compound of formula I | 99.3% | 100.2% | 99.0% | 99.6% |
| | Content of related substances (total impurity) | Not detected | Not detected | Not detected | Not detected |
| High temperature (60 ± 2°C) | Content of compound of formula I | 99.3% | 100.5% | 98.9% | 100.4% |
| | Content of related substances (total impurity) | Not detected | Not detected | Not detected | 0.17% |
| High humidity (92.5% RH) | Content of compound of formula I | 99.3% | 100.6% | 99.1% | 100.7% |
| | Content of related substances (total impurity) | Not detected | Not detected | Not detected | Not detected |

### Example 11: Accelerated stability test of 0.5% (w/w) specification of formulation A2

**Table 16**

| Test item | Placement condition | Day 0 | Month 1 | Month 2 | Month 3 |
|---|---|---|---|---|---|
| Property | 40 ± 2°C, 75 ± 5%RH | Yellow transpare nt oily liquid | Yellow transpare nt oily liquid | Yellow transpare nt oily liquid | Yellow transpare nt oily liquid |
| Content of compound of formula I | | 99.3% | 100.0% | 100.0% | 99.3% |
| Related substance (total impurity content) | | ND | ND | 0.06% | ND |

Experiments have shown that formulation A2, with specifications of 2% (w/w) and 0.5% (w/w), exhibits good stability under conditions of illumination, high temperature, and high humidity.

### Example 12: Preparation of a pharmaceutical composition containing 2% (w/w) of the compound of formula II

**Table 17**

| **Component** | **Formulation 1** | **Formulation 2** | **Formulation 3** | **Formulation 4** | **Formulation 5** | **Formulation 6** |
|---|---|---|---|---|---|---|
| **Compound of formula II** | 2.00% | 2.00% | 2.00% | 2.00% | 2.00% | 2.00% |
| **Polyethylene glycol 400** | 40.00% | 40.00% | 40.00% | 40.00% | 40.00% | 40.00% |
| **Diethylene glycol monoethyl ether** | 25.00% | 25.00% | 25.00% | 25.00% | 10.00% | 25.00% |
| **Propyl gallate** | 26.00% | 26.00% | 32.94% | 26.00% | 42.94% | 32.96% |
| **Ethanol** | - | - | - | - | - | - |
| **Azone** | - | - | - | - | - | - |
| **Oleic acid** | 7.00% | - | - | - | - | - |
| **Diisopropyl adipate** | - | 7.00% | - | - | - | - |
| **Menthol** | - | - | 0.06% | - | 0.06% | - |
| ***N-*Methylpyrrolidone** | - | - | - | 7.00% | - | - |
| **Dimethyl sulfoxide** | - | - | - | - | 5.00% | - |
| **Imidurea** | - | - | - | - | - | 0.04% |
| **Total** | 100.00% | 100.00% | 100.00% | 100.00% | 100.00% | 100.00% |

Preparation method: Except for the difference in active ingredients, the preparation was carried out according to the preparation method of Example 2.

### Example 13: Preparation of a pharmaceutical composition containing 2% (w/w) of the compound of formula II

**Table 18**

| **Component** | **Formulation 7** | **Formulation 8** | **Formulation 9** |
|---|---|---|---|
| **Compound of formula II** | 2.00% | 2.00% | 2.00% |
| **Polyethylene glycol 400** | 39.60% | 40.00% | 40.00% |
| **Diethylene glycol monoethyl ether** | 24.75% | 10.00% | 10.00% |
| **Propylene glycol** | 9.90% | 36.00% | 37.94% |
| **Ethanol** | 19.80% | - | - |
| **Azone** | 3.95% | - | - |
| **Oleic acid** | - | 7.00% | - |
| **Menthol** | - | - | 0.06% |
| **Dimethyl sulfoxide** | - | 5.00% | 10.00% |
| **Total** | 100.00% | 100.00% | 100.00% |

Preparation method: Except for the difference in active ingredients, the preparation was carried out according to the preparation method of Example 3.

### Example 14: In vitro penetration test (IVPT)

According to the experimental steps of Example 4, six formulations 3, 4, 5, 7, 8, and 9 were respectively tested for the average cumulative permeation rate *in vitro.* The test results are shown in Tables 19 and 20:

**Table 19: Average cumulative permeation rates at each sampling time point in an in vitro permeation test of formulations 3, 4, and 5 with 2% (w/w) specifications**

| **Formulation** | **Sampling time point (h)** | **Average cumulative permeation rate%** |
|---|---|---|
| **3** | 18 | 0.016% |
| | 24 | 0.033% |
| | 36 | 0.058% |
| | 48 | 0.062% |
| **4** | 18 | 0.074% |
| | 24 | 0.141% |
| | 36 | 0.285% |
| | 48 | 0.332% |
| **5** | 18 | 0.067% |
| | 24 | 0.115% |
| | 36 | 0.211% |
| | 48 | 0.237% |

According to the results in Table 19, it can be seen that the average cumulative permeation rates at each sampling time point in the *in vitro* permeation test of formulation 3, formulation 4, and formulation 5 are in the order of 4 > 5 > 3 from large to small.

**Table 20: Average cumulative permeation rates at each sampling time point in an in vitro permeation test of formulations 7, 8, and 9 with 2% (w/w) specifications**

| **Formulation** | **Sampling time point (h)** | **Average cumulative permeation rate%** |
|---|---|---|
| **7** | 18 | 0.015% |
| | 24 | 0.027% |
| | 36 | 0.092% |
| | 48 | 0.154% |
| **8** | 18 | 0.352% |
| | 24 | 0.498% |
| | 36 | 0.954% |
| | 48 | 1.097% |
| **9** | 18 | 0.012% |
| | 24 | 0.020% |
| | 36 | 0.069% |
| | 48 | 0.084% |

According to the results in Table 20, it can be seen that the average cumulative permeation rate of formulation 8 in the *in vitro* permeation test within 48 hours is significantly higher than that of formulation 9 and formulation 7.

### Example 15: Preparation process of 2% (w/w) specification of formulation 8

### (1) Prescription composition of 2% (w/w) specification of formulation 8

**Table 21**

| **Material** | **Ratio% (w/w)** | **Prescription amount (g)** |
|---|---|---|
| Compound of formula II | 2 | 37 |
| Polyethylene glycol 400 | 40 | 740 |
| Diethylene glycol monoethyl ether | 10 | 185 |
| Propylene glycol | 36 | 666 |
| Oleic acid | 7 | 129.5 |
| Dimethyl sulfoxide | 5 | 92.5 |
| Total | 100 | 1850 |

### (2) Preparation steps

According to the prescription composition of the 2% (w/w) specification of formulation 8, the preparation was completed with reference to the preparation steps of Example 6.

### Example 16: Preparation process of 0.5% (w/w) specification of formulation 8

### (1) Prescription composition of 0.5% (w/w) specification of formulation 8

**Table 22**

| **Material** | **Ratio% (w/w)** | **Prescription amount (g)** |
|---|---|---|
| Compound of formula II | 0.5 | 13.25 |
| Polyethylene glycol 400 | 41.5 | 1099.75 |
| Diethylene glycol monoethyl ether | 10.0 | 265.0 |
| Propylene glycol | 36.0 | 954.0 |
| Oleic acid | 7.0 | 185.5 |
| Dimethyl sulfoxide | 5.0 | 132.5 |
| **Total** | 100.0 | 2650 |

### (2) Preparation steps

According to the prescription composition of the 0.5% (w/w) specification of formulation 8, the preparation was completed with reference to the preparation steps of Example 6.

### Example 17: Stability study of 2% (w/w) specification of formulation 8

**Table 23**

| Placement condition | Test item | Day 0 | Day 5 | Day 10 | Day 30 |
|---|---|---|---|---|---|
| Illumination (4500 Lx ± 500 Lx) | Content of compound of formula II | 99.5% | 100.1% | 99.2% | 99.8% |
| | Content of related substances (total impurity) | Not detected | Not detected | Not detected | Not detected |
| **High temperature** (60 ± 2°C) | Content of compound of formula II | 98.5% | 101.2% | 100.3% | 101.0% |
| | Content of related substances (total impurity) | Not detected | Not detected | Not detected | Not detected |
| High humidity (92.5% RH) | Content of compound of formula II | 98.5% | 101.1% | 100.2% | 101.1% |
| | Content of related substances (total impurity) | Not detected | Not detected | Not detected | Not detected |

### Example 18: Accelerated stability test of 2% (w/w) specification of formulation 8

**Table 24**

| Test item | Placement condition | Day 0 | Month 1 | Month 2 | Month 3 |
|---|---|---|---|---|---|
| Property | 40 ± 2°C, 75 ± 5%RH | Yellow transpare nt oily liquid | Yellow transpare nt oily liquid | Yellow transparen t oily liquid | Yellow transpare nt oily liquid |
| Content of compound of formula II | | 99.5% | 100.0% | 100.0% | 99.6% |
| Related substance (total impurity content) | | Not detected | Not detected | 0.05% | Not detected |

### Example 19: Stability study of 0.5% (w/w) specification of formulation 8

**Table 25**

| Placement condition | Test item | Day 0 | Day 5 | Day 10 | Day 30 |
|---|---|---|---|---|---|
| Illumination (4500 Lx ± 500 Lx) | Content of compound of formula II | 99.5% | 100.0% | 99.3% | 99.8% |
| | Content of related substances (total impurity) | Not detected | Not detected | Not detected | Not detected |
| **High temperature** (60 ± 2°C) | Content of compound of formula II | 99.5% | 100.1% | 99.2% | 100.0% |
| | Content of related substances (total impurity) | Not detected | Not detected | Not detected | 0.13% |
| High humidity (92.5% RH) | Content of compound of formula II | 99.6% | 100.1% | 99.5% | 100.2% |
| | Content of related substances (total impurity) | Not detected | Not detected | Not detected | Not detected |

### Example 20: Accelerated stability test of 0.5% (w/w) specification of formulation 8

**Table 26**

| Test item | Placement condition | Day 0 | Month 1 | Month 2 | Month 3 |
|---|---|---|---|---|---|
| Property | 40 ± 2°C, 75 ± 5%RH | Yellow transpare nt oily liquid | Yellow transpare nt oily liquid | Yellow transpare nt oily liquid | Yellow transpare nt oily liquid |
| Content of compound of formula II | | 99.6% | 100.1% | 100.0% | 99.5% |
| Related substance (total impurity content) | | ND | ND | 0.05% | ND |

Experiments have shown that formulation 8, with specifications of 2% (w/w) and 0.5% (w/w), exhibits good stability under conditions of illumination, high temperature, and high humidity.

## Claims

1. A pharmaceutical composition comprising a JAK inhibitor, wherein the pharmaceutical composition comprises the following components in mass fraction: 0.1% to 3% of a compound of formula I or a compound of formula II or a pharmaceutically acceptable salt thereof, 35% to 93% of a solvent, and 5% to 63% of a transdermal enhancer;
the solvent is selected from one or more of water, glycerol, polyethylene glycol 400, dimethyl sulfoxide, ethyl acetate, propylene glycol, or ethanol;
the transdermal enhancer is selected from one or more of diethylene glycol monoethyl ether, azone, urea, oleic acid, diisopropyl adipate, menthol, N-methylpyrrolidone, imidurea, propyl gallate, isopropyl myristate, cyclodextrin, sodium dodecyl sulfate, polyoxyethylene lauryl ether, polyethylene glycol hexadecyl ether, Tween 20, Tween 40, Tween 60, Tween 80, Span 20, Span 40, Span 60, Span 80, Span 85, deoxycholate, glycocholate, glyceryl monocaprylate, caprylic/capric mono- and diglycerides, propylene glycol monolaurate, propylene glycol monocaprylate, or glyceryl behenate;

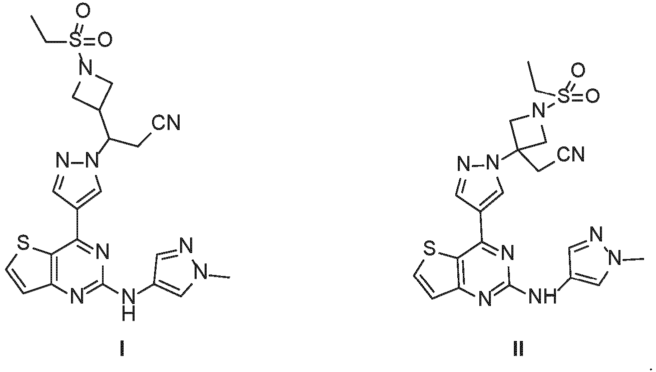

2. The pharmaceutical composition according to claim 1, wherein the compound of formula I or the compound of formula II or the pharmaceutically acceptable salt thereof has a mass fraction of 0.3% to 2.5%;
and/or, the solvent has a mass fraction of 38% to 90%; preferably 40% to 88%, for example, 40%, 45%, 69.3%, 81%, 82.5%, or 87.94%;
and/or, the transdermal enhancer has a mass fraction of 8% to 60%; preferably 10% to 58%, for example, 10.06%, 17%, 28.7%, 53%, or 58%;
and/or, the solvent is selected from one or more of polyethylene glycol 400, dimethyl sulfoxide, propylene glycol, or ethanol, for example, polyethylene glycol 400; a mixed solvent of polyethylene glycol 400 and dimethyl sulfoxide; a mixed solvent of polyethylene glycol 400, propylene glycol, and ethanol; or a mixed solvent of polyethylene glycol 400, propylene glycol, and dimethyl sulfoxide;
and/or, the transdermal enhancer is selected from one or more of diethylene glycol monoethyl ether, azone, oleic acid, diisopropyl adipate, menthol, N-methylpyrrolidone, imidurea, or propyl gallate, for example, a mixed transdermal enhancer of diethylene glycol monoethyl ether, propyl gallate, and oleic acid; a mixed transdermal enhancer of diethylene glycol monoethyl ether, propyl gallate, and diisopropyl adipate; a mixed transdermal enhancer of diethylene glycol monoethyl ether, propyl gallate, and menthol; a mixed transdermal enhancer of diethylene glycol monoethyl ether, propyl gallate, and N-methylpyrrolidone; a mixed transdermal enhancer of diethylene glycol monoethyl ether, propyl gallate, and imidurea; a mixed transdermal enhancer of diethylene glycol monoethyl ether and azone; a mixed transdermal enhancer of diethylene glycol monoethyl ether and oleic acid; or a mixed transdermal enhancer of diethylene glycol monoethyl ether and menthol.

3. The pharmaceutical composition according to claim 2, wherein the compound of formula I or the compound of formula II or the pharmaceutically acceptable salt thereof has a mass fraction of 0.5% to 2%, for example, 0.5% or 2%;
and/or, the solvent has a mass fraction of 78% to 85%, for example, 81% or 82.5%;
and/or, the transdermal enhancer has a mass fraction of 15% to 19%, for example, 17%;
and/or, the solvent is selected from one or more of polyethylene glycol 400, dimethyl sulfoxide, or propylene glycol, for example, a mixed solvent of polyethylene glycol 400, dimethyl sulfoxide, and propylene glycol;
and/or, the transdermal enhancer is selected from one or two of diethylene glycol monoethyl ether or oleic acid, for example, a mixed transdermal enhancer of diethylene glycol monoethyl ether and oleic acid.

4. The pharmaceutical composition according to claim 2, wherein when the solvent is a mixed solvent of polyethylene glycol 400 and dimethyl sulfoxide, the mass ratio of polyethylene glycol 400 to dimethyl sulfoxide is from 6:1 to 10:1, preferably from 7:1 to 9:1, for example, 8:1;
and/or, when the solvent is a mixed solvent of polyethylene glycol 400, propylene glycol, and ethanol, the mass ratio of polyethylene glycol 400 to propylene glycol is from 2:1 to 6:1, preferably from 3:1 to 5:1, for example, 4:1; the mass ratio of propylene glycol to ethanol is from 1:1 to 1:3, preferably from 1:1.5 to 1:2.5, for example, 1:2;
and/or, when the solvent is a mixed solvent of polyethylene glycol 400, propylene glycol, and dimethyl sulfoxide, the mass ratio of polyethylene glycol 400 to dimethyl sulfoxide is from 3:1 to 10:1, preferably from 6:1 to 10:1, more preferably from 7:1 to 9:1, for example, 8:1 or 8.3:1; the mass ratio of propylene glycol to dimethyl sulfoxide is from 3:1 to 9:1, preferably from 5:1 to 9:1, more preferably from 6:1 to 8:1, for example, 7.2:1;
and/or, when the transdermal enhancer is a mixed transdermal enhancer of diethylene glycol monoethyl ether, propyl gallate, and oleic acid, the mass ratio of diethylene glycol monoethyl ether to oleic acid is from 2:1 to 5:1, preferably from 3:1 to 4:1, for example, 3.57:1; the mass ratio of propyl gallate to oleic acid is from 2:1 to 5:1, preferably from 3:1 to 4:1, for example, 3.71:1;
and/or, when the transdermal enhancer is a mixed transdermal enhancer of diethylene glycol monoethyl ether, propyl gallate, and diisopropyl adipate, the mass ratio of diethylene glycol monoethyl ether to diisopropyl adipate is from 2:1 to 5:1, preferably from 3:1 to 4:1, for example, 3.57:1; the mass ratio of propyl gallate to diisopropyl adipate is from 2:1 to 5:1, preferably from 3:1 to 4:1, for example, 3.71:1;
and/or, when the transdermal enhancer is a mixed transdermal enhancer of diethylene glycol monoethyl ether, propyl gallate, and menthol, the mass ratio of diethylene glycol monoethyl ether to menthol is from 150:1 to 430:1, preferably from 160:1 to 420:1, for example, 166.67:1 or 416.67:1; the mass ratio of propyl gallate to menthol is from 530:1 to 730:1, preferably from 540:1 to 720:1, for example, 549:1 or 715.67:1;
and/or, when the transdermal enhancer is a mixed transdermal enhancer of diethylene glycol monoethyl ether, propyl gallate, and *N*-methylpyrrolidone, the mass ratio of diethylene glycol monoethyl ether to *N*-methylpyrrolidone is from 2:1 to 5:1, preferably from 3:1 to 4:1, for example, 3.57:1; the mass ratio of propyl gallate to *N*-methylpyrrolidone is from 2:1 to 5:1, more preferably from 3:1 to 4:1, for example, 3.71:1;
and/or, when the transdermal enhancer is a mixed transdermal enhancer of diethylene glycol monoethyl ether, propyl gallate, and imidurea, the mass ratio of diethylene glycol monoethyl ether to imidurea is from 610:1 to 640:1, preferably from 620:1 to 630:1, for example, 625:1; the mass ratio of propyl gallate to imidurea is from 810:1 to 840:1, preferably from 820:1 to 830:1, for example, 824:1;
and/or, when the transdermal enhancer is a mixed transdermal enhancer of diethylene glycol monoethyl ether and azone, the mass ratio of diethylene glycol monoethyl ether to azone is from 4:1 to 8:1, preferably from 5:1 to 7:1, for example, 6.27:1;
and/or, when the transdermal enhancer is a mixed transdermal enhancer of diethylene glycol monoethyl ether and oleic acid, the mass ratio of diethylene glycol monoethyl ether to oleic acid is from 1.2:1 to 1.6:1, preferably from 1.3:1 to 1.5:1, for example, 1.43:1;
and/or, when the transdermal enhancer is a mixed transdermal enhancer of diethylene glycol monoethyl ether and menthol, the mass ratio of diethylene glycol monoethyl ether to menthol is from 150:1 to 180:1, preferably from 160:1 to 170:1, for example, 166.67:1.

5. The pharmaceutical composition according to any one of claims 1 to 4, wherein the pharmaceutical composition comprising the JAK inhibitor is composed of the compound of formula I or the compound of formula II or the pharmaceutically acceptable salt thereof, the solvent, and the transdermal enhancer.

6. The pharmaceutical composition according to claim 5, wherein the pharmaceutical composition comprising the JAK inhibitor is composed of components shown in any one of the following schemes:
scheme 1: 2% of the compound of formula I or the compound of formula II or the pharmaceutically acceptable salt thereof, 40% of polyethylene glycol 400, 25% of diethylene glycol monoethyl ether, 26% of propyl gallate, and 7% of oleic acid;
scheme 2: 2% of the compound of formula I or the compound of formula II or the pharmaceutically acceptable salt thereof, 40% of polyethylene glycol 400, 25% of diethylene glycol monoethyl ether, 26% of propyl gallate, and 7% of diisopropyl adipate;
scheme 3: 2% of the compound of formula I or the compound of formula II or the pharmaceutically acceptable salt thereof, 40% of polyethylene glycol 400, 25% of diethylene glycol monoethyl ether, 32.94% of propyl gallate, and 0.06% of menthol;
scheme 4: 2% of the compound of formula I or the compound of formula II or the pharmaceutically acceptable salt thereof, 40% of polyethylene glycol 400, 25% of diethylene glycol monoethyl ether, 26% of propyl gallate, and 7% of N-methylpyrrolidone;
scheme 5: 2% of the compound of formula I or the compound of formula II or the pharmaceutically acceptable salt thereof, 40% of polyethylene glycol 400, 10% of diethylene glycol monoethyl ether, 42.94% of propyl gallate, 0.06% of menthol, and 5% of dimethyl sulfoxide;
scheme 6: 2% of the compound of formula I or the compound of formula II or the pharmaceutically acceptable salt thereof, 40% of polyethylene glycol 400, 25% of diethylene glycol monoethyl ether, 32.96% of propyl gallate, and 0.04% of imidurea;
scheme 7: 2% of the compound of formula I or the compound of formula II or the pharmaceutically acceptable salt thereof, 39.6% of polyethylene glycol 400, 24.75% of diethylene glycol monoethyl ether, 9.9% of propylene glycol, 19.8% of ethanol, and 3.95% of azone;
scheme 8: 2% of the compound of formula I or the compound of formula II or the pharmaceutically acceptable salt thereof, 40% of polyethylene glycol 400, 10% of diethylene glycol monoethyl ether, 36% of propylene glycol, 7% of oleic acid, and 5% of dimethyl sulfoxide;
scheme 9: 0.5% of the compound of formula I or the compound of formula II or the pharmaceutically acceptable salt thereof, 41.5% of polyethylene glycol 400, 10% of diethylene glycol monoethyl ether, 36% of propylene glycol, 7% of oleic acid, and 5% of dimethyl sulfoxide;
scheme 10: 2% of the compound of formula I or the compound of formula II or the pharmaceutically acceptable salt thereof, 40% of polyethylene glycol 400, 10% of diethylene glycol monoethyl ether, 37.94% of propylene glycol, 0.06% of menthol, and 10% of dimethyl sulfoxide;
percentages above are all mass percentages.

7. The pharmaceutical composition according to claim 1, wherein the pharmaceutical composition is an ointment, a gel, a solution, a spray, or a suspension, preferably a solution.

8. A preparation method for the pharmaceutical composition according to any one of claims 1 to 7, wherein the preparation method comprises the following step: mixing the compound of formula I or the compound of formula II or the pharmaceutically acceptable salt thereof, the solvent, and the transdermal enhancer.

9. The preparation method according to claim 8, wherein the preparation method comprises the following steps: after mixing the solvent and the transdermal enhancer, dissolving the compound of formula I or the compound of formula II or the pharmaceutically acceptable salt thereof therein, to obtain the pharmaceutical composition comprising the JAK inhibitor.

10. The preparation method according to claim 9, wherein the compound of formula I or the compound of formula II or the pharmaceutically acceptable salt thereof is the compound of formula I or the compound of formula II;
and/or, the dissolving is by means of ultrasound, heating, or a combination of both; the heating is preferably water bath heating; and the heating is preferably at a temperature of 60°C to 70°C.

11. The preparation method according to claim 8, wherein when the pharmaceutical composition comprises dimethyl sulfoxide, the preparation method for the pharmaceutical composition comprises the following steps:
(1) mixing the dimethyl sulfoxide and the compound of formula I or the compound of formula II or the pharmaceutically acceptable salt thereof in the components to obtain a mixture;
(2) mixing all components except the dimethyl sulfoxide and the compound of formula I or the compound of formula II or the pharmaceutically acceptable salt thereof in the components to obtain a mixture;
(3) mixing the mixture of step (1) with the mixture of step (2) to obtain the pharmaceutical composition comprising the JAK inhibitor.

12. The preparation method according to claim 11, wherein the pharmaceutical composition is the pharmaceutical composition shown in the scheme 5, the scheme 8, the scheme 9, or the scheme 10;
and/or, the compound of formula I or the compound of formula II or the pharmaceutically acceptable salt thereof is the compound of formula I or the compound of formula II;
and/or, in step (3), the mixing is adding the mixture of step (2) to the mixture of step (1);
and/or, the mixture of step (1) and the mixture of step (2) are each mixed homogeneously by stirring; the stirring is preferably at a rotation speed of 1500 rpm to 2500 rpm, for example, 2000 rmp.

13. A topical liniment comprising the pharmaceutical composition according to any one of claims 1 to 7.

14. A use of the pharmaceutical composition according to any one of claims 1 to 7 or the topical liniment according to claim 13 for preventing, alleviating, or treating skin autoimmune diseases related to JAK kinase; the skin autoimmune disease is preferably selected from one or more of alopecia areata, atopic dermatitis, or psoriasis.
